# EUROPEAN PATENT APPLICATION

(11) **EP 2 213 332 A1**
(43) Date of publication of application: **04.08.2010**
(21) Application number: 08838029.0
(22) Date of filing: 09.10.2008
(51) Int. Cl.: A61N 1/40, A61N 1/30

(54) **DEVICE FOR TRANSDERMIC TRANMISSION AND TRANSFER OF HEAT TO INTERNAL TISSUES BY RADIOFREQUENCY**

(30) Priority: 11.10.2007 ES 200702682
(71) Applicant: Instituto De Terapias Biológicas, S.l., 31119 Imarcoain (Navarra) (ES)
(72) Inventor: GARCÍA ARMENDÁRIZ, Francisco, E-31119 Imarcoain (Navarra) (ES)
(86) International application number: PCT/ES2008/000634
(87) International publication number: WO 2009/047379

(57) **Abstract**

**DEVICE FOR TRANSDERMIC TRANSMISSION AND TRANSFER OF HEAT TO INTERNAL TISSUES BY RADIOFREQUENCY**

Specifically designed to combine means for carrying out electroporation and diathermia/hyperthermia in the same device; said device comprises a casing (1) wherein microprocessors or microcontrollers (2) and (3) are installed with internal firmware programmed to generate waves of the type used in both methods through each of the former. An interface (4) is used for selecting the technique to be applied, the application time, and in the case of diathermia, the exposure power or temperature. Thus, the said microprocessors (2) and (3), generate radiofrequency waves suited to each treatment, said waves are transmitted through connectors (8-11), one of which is intended for connecting anatomical headpieces (13), used for either electroporation or diathermia/hyperthermia treatments, and the other connector is for connecting active plates (9) for diathermia/hyperthermia treatments.

## Description

### OBJECT OF THE INVENTION

The present specification refers to a Patent Application regarding a device specifically designed to carry out in the same device diverse techniques, including transdermic transmission of active principles as well as transferring heat to internal tissues, specifically the mesoporation or electroporation technique and diathermia or hyperthermia.

### FIELD OF THE INVENTION

This invention is framed into the devices used in aesthetic medicine, physiotherapy, sports medicine, and similar.

### BACKGROUND OF THE INVENTION

Currently, the electroporation or transdermic transmission is one the best methods developed for the introduction of active principles via epidermis. Its use in biotechnological projects for cellular ADN introduction, supports the possibilities of application in different branches of medicine, where it is found the aesthetic medicine and physiotherapy.

Said technique is an alternative to the traditional routes of administration (oral, intramuscular and intravenous) of active principles and medicines. Said technique also offers advantages as easy access to the skin, of the solution in specific areas of treatment, and avoids collateral effects of oral and parenteral routes of administration, as well as, the lack of use of needles and its consequent aggressive mechanism (injection-pain), due to their mandatory use as a general guideline in mesotherapy treatments and infiltrations.

The transdermic transmission or electroporation equipments already exist in the market and its development is based on the need of a non aggressive system to introduce active principles below the stratum corneum, which results very important in Research and Development Departments (cellular, pharmacology) and also for its use in treatments requiring the supply of active principles into the body. It is an alternative to injections (needles).

The electroporation is the transdermic penetrarion of any active principle (ionized or not), having any kind of water-soluble or liposoluble molecules, carried by a liposome complex, liquid solution or any proper fluid.

So, the electroporation is a revolutionary technique capable of increase the uptake ability of the cells, since it eases the pass of substances through the cellular tissue. That way the opening of the cellular tissue channels (proteic channels and lipid channels) is inducted, as well as the cellular pores which become more likely to uptake substances.

Consequently, this technique results of special interest for those professionals belonging to aesthetic medicine, physiotherapy, sports medicine, rehabilitation and research, because is able to produce the same effects of mesotherapy or other conventional techniques of infiltrations, without needles, without pain and without any side effects different than those generated by product used itself or the patient health.

Equally known the hyperthermia equipments are, or better known as diathermia equipments. Basically they are used in rehabilitation, where its main characteristic relies on the transfer of heat within the organism with no change in the temperature of the skin or epidermis (heating of internal tissues without side effects). These equipments have been used in the treatment of internal injuries because they increase the blood flow around the injured zone (oxygenating the treated area), helping the fluidization of colloids and accumulated fats which means their elimination, increasing the metabolic reactions, relaxing the muscular fibre due to its antispasmodic properties, boosting the production of natural substances of the organism such as collagen and improving the reduction of pain symptoms by the stimulation of the neurotransmitters and production of endorphins.

However, the applicant does not acknowledge the existence of any equipment capable to carry out both techniques with only one device.

### DESCRIPTION OF THE INVENTION

The device for transdermic transmission and transfer of heat to internal tissues which the invention proposes solves in a total satisfactory way the problems above described, gathering in only one device mechanisms to put into practice both techniques previously described, resulting of great utility since the diathermia technique could be used as a preparation for the electroporation.

In order to carry out what stated above, the equipment proposed is composed by a casing, where the corresponding user interface is placed to use the device. The system core hosts a pair of boards or circuits. Each one has its own microprocessor and own firmware to generate the waves needed to carry out properly both techniques.

In a more specific way, the device includes an internal firmware with parameters of use, such as wavelength, intensity, radiation frequency, etc, already set which could be selected by the user as preset programmes. It is always advised to allow the user only modifies parameters such as temperature (exposure power) and time of treatment. Eventually and according to the type of user employing the device i.e. research personnel, the device could be programmed for a total modification of parameters.

Using the mentioned user interface, it will be possible to select the technique or techniques the user is wishing to use. In the case of diathermia, the user should control the parameters previously said including preset programs, time and temperature of the treatment, while using the electroporation program the user will only need to select the preset program and the time of exposure.

The casing will have a pair of connectors; one of them will be used for the connection of a single anatomic headpiece which could receive signals from either diathermia circuit or the electroporation circuit, to allow apply said techniques on the body areas to treat, and the second connector will be only associated to the diathermia circuit, allowing the connection of active plates, which could be either monopolar or bipolar plates, for the treatment of bigger areas.

The equipment has been provided with an optional electrostimulation module in order to produce electric pulses for the muscular treatment by stimulation in specific areas; in this case the casing will also have an output for the corresponding electrodes. The equipment also includes the proper interface with the same parameters of time and intensity for the electric impulse.

Finally, it is important to point out the obvious fact, that the microprocessors trough the corresponding waves of treatment are generated could be perfectly substituted by circuits provided with knobs, being an equivalent solution but analogical.

### DESCRIPTION OF THE DRAWINGS

In order to complement the description being made and to permit a better understanding of the features of the invention, the present specification is accompanied by a set of drawings showing the following with an illustrative and non-limiting character.

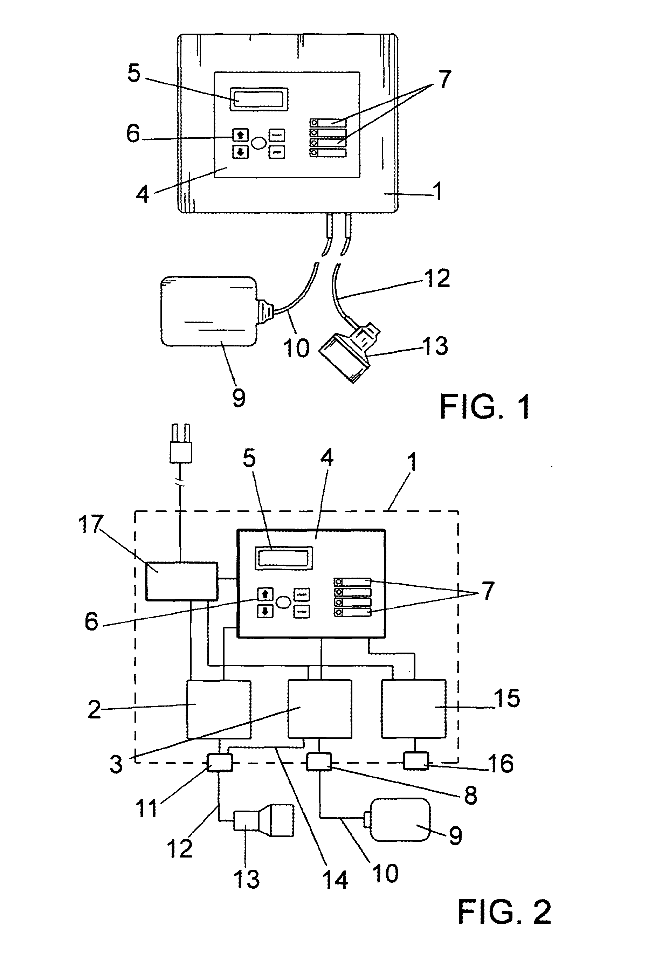

Figure No. 1 - Shows, according to a perspective schematic representation the object of the invention, a device for transdermic transmission and transfer of heat to internal tissues by radiofrequency.
Figure No. 2 - Shows, a schematic representation of the different elements composing the device object of the invention.

### PREFERRED EMBODIMENT OF THE INVENTION

Following these figures, it may be observed how the device proposed is composed by a casing (1), wherein the prototype example adopts a rectangular prismatic shape which however, could adopt any other shape according to specific and different design lines, said casing (1) is intended to host the correspondent circuitry where at least a pair of microprocessors or microcontrollers will be placed (2) and (3) with a proper programming firmware to generate radiofrequency waves in accord with the two techniques intended to combine into the equipment, which are the electroporation and diathermia/hyperthermia.

Most of the parameters required to generate the treatment waves - such as intensity, frequency, wavelength, etc. - will be already gathered and preset into the said firmware as preset programs, in addition to these, only temperature and time treatment would be selectable to modify.

In order to control said values, the microprocessors (2) and (3) have been linked to the user interface (4), wherein is place a display (5), a keypad (6) and some lights (7) to control the device.

To the output of one of the microprocessors (3), especially the one intended to include the firmware generator of radiofrequency waves for diathermia/hyperthermia treatments, will have a connector (8) in order to plug the active plate (9) trough the corresponding cable (10), said active plate (9) could be either monopolar or bipolar type.

In the same way, to the microcontroller (2), intended to generate the radiofrequency waves for electroporation treatments, is linked a connector (11) in order to plug the corresponding cables (12) of different anatomic headpieces (13), of different sizes depending of the area of treatment, having the special feature of being able to connect electrically (14) it to the microprocessor (3), this is done in order to allow said anatomic headpieces to be used in either electroporation and diatermia/hipertermia techniques.

Just as mentioned before, additionally the device could include an electro stimulation module (15), which uses direct current to produce electric pulses for muscular treatment by stimulation in specific areas, said module (15) which will be also linked to an output (16) to plug the corresponding electrodes, and to the interface (4) through could be selected the time and intensity of the electric impulses.

Finally, as a standard the device will be equipped with the proper electric power supply and the tensions adapters (17) to supply the different internal devices/pieces into the equipment.

According to this structure, the device functioning is the following one:
Once the device is plugged, trough the selector or keypad (6) chooses the type of technique to use (diathermia or electroporation), since it was expected that both techniques could offer different preset programs of treatment based on the most suitable parameters of intensity, frequency and wavelength emitted. Next, the duration time of treatment is selected, and if the diathermia mode was selected then the power and temperature of treatment should be set.
Once followed these step, the corresponding light (7) will shine, informing there is emission, so the active plate (9) or the headpiece (13) will be ready to treat the patient.

## Claims

1. ^{a}- Device for transdermic transmission and transfer of heat to internal tissues by radiofrequency, which having for ultimate goal combine in the same device the mechanisms needed to carry out electroporation techniques and diathermia/hyperthermia techniques, said device is **characterized by** comprising a casing (1) wherein core there are at least a pair of microprocessors o microcontrollers (2) and (3), with an internal programmed firmware suitable to generate through each of them radiofrequency waves of the type used in electroporation and diathermia/hyperthermia treatments, said microprocessors (2) and (3) are linked to an interface (4) to control the device, said microprocessors have been associated to at least a pair of connectors (8-11), one of them will be used to plug the anatomic headpieces (13) for either electroporation or diathermia/hyperthermia treatments, the second one will allow the connection of active plates (9) for diathermia/hyperthermia treatments.

2. ^{a}.- Device for transdermic transmission and transfer of heat to internal tissues by radiofrequency, according to claim 1^{a}, said device is **characterized** because optionally the device could incorporate an electro stimulation module (15), associated to an output (16) to plug the corresponding electrodes, and to plug to the programming interface (4).
